(19) Europäisches Patentamt
European Patent Office
Office européen des brevets

(11) **EP 3 102 111 B1**

(12) **EUROPEAN PATENT SPECIFICATION**

(45) Date of publication and mention
of the grant of the patent:
**03.04.2019 Bulletin 2019/14**

(51) Int Cl.:
*A61B 6/03* (2006.01)     *A61N 5/00* (2006.01)
*G06K 9/60* (2006.01)     *G06K 9/78* (2006.01)
*G06K 9/80* (2006.01)     *A61B 6/00* (2006.01)
*G06T 11/00* (2006.01)

(21) Application number: **15746947.9**

(22) Date of filing: **05.02.2015**

(86) International application number:
**PCT/AU2015/000058**

(87) International publication number:
**WO 2015/117187 (13.08.2015 Gazette 2015/32)**

(54) **OPTIMISED 4D CONE BEAM COMPUTED TOMOGRAPHY PROJECTION ALLOCATION**

OPTIMIERTE 4D-COMPUTERTOMOGRAFIEPROJEKTIONSZUTEILUNG

ATTRIBUTION DE PROJECTION PAR TOMODENSITOMÉTRIE 4D OPTIMISÉE A FAISCEAU CONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.02.2014 AU 2014900354**

(43) Date of publication of application:
**14.12.2016 Bulletin 2016/50**

(73) Proprietor: **The University of Sydney New South Wales 2006 (AU)**

(72) Inventors:
• **O'BRIEN, Ricky**
  **Rozelle, New South Wales 2039 (AU)**
• **KEALL, Paul**
  **Greenwich, New South Wales 2065 (AU)**

(74) Representative: **Viering, Jentschura & Partner mbB**
  **Patent- und Rechtsanwälte**
  **Am Brauhaus 8**
  **01099 Dresden (DE)**

(56) References cited:
**WO-A2-2013/016689**

• **LI TIANFANG ET AL: "Enhanced 4D cone-beam CT with inter-phase motion model", MEDICAL PHYSICS, AIP, MELVILLE, NY, US, vol. 34, no. 9, 27 August 2007 (2007-08-27), pages 3688-3695, XP012103571, ISSN: 0094-2405, DOI: 10.1118/1.2767144**
• **O'BRIEN, R. ET AL.: 'Optimizing 4D Cone Beam Computed Tomography Acquisition by Varying the Gantry Velocity and Projection Time Interval' PHYSICS IN MEDICINE AND BIOLOGY vol. 58, no. 6, 2013, pages 1705 - 1723, XP020242413**
• **BERGNER, F. ET AL.: 'Autoadaptive Phase-Correlated (AAPC) Reconstruction for 4D CBCT' MEDICAL PHYSICS, [Online] vol. 36, no. 12, 2009, pages 5695 - 5706, XP012129843 Retrieved from the Internet: <URL:http://www.imp.uni-erlangen.de/imp_info/mitarbeiter/mare/AAPC-4DCBCT.pdf> [retrieved on 2015-03-16]**

**Description**

**FIELD OF THE INVENTION**

The present invention relates to the field of cone beam computed tomography imaging and, in particular, relates to optimizing four dimensional cone beam computed tomography (4DCBCT) images of the thorax and upper abdomen and any tumours in these regions.

**REFERENCES**

Abdelnour A F, Nehmeh S A, Pan T, Humm J L, Vernon P, Schoder H, Rosenzweig K E, Mageras G S, Yorke E, Larson S M & Erdi Y E 2007 Phase and amplitude binning for 4D-CT imaging Phys Med Biol 52(12), 3515-29.

Chen G H, Tang J & Leng S 2008 Prior image constrained compressed sensing (PICCS): a method to accurately reconstruct dynamic CT images from highly undersampled projection data sets Med Phys 35(2), 660-3.

Cooper B J, O'Brien R T, Balik S, Hugo G D & Keall P J 2013 Respiratory triggered 4D cone-beam computed tomography: a novel method to reduce imaging dose Med Phys 40(4), 041901.

Dietrich L, Jetter S, Tucking T, Nill S & Oelfke U 2006 Linac-integrated 4D cone beam CT: first experimental results Phys Med Biol 51(11), 2939-52.

Fast M, Wisotzky E, Oelfke U & Nill S 2013 Actively Triggered 4d Cone-Beam CT Acquisition Medical Physics 40(9), 0.

Feldkamp L A, Davis L C & Kress J W 1984 Practical Cone-Beam Algorithm Journal of the Optical Society of America a-Optics Image Science and Vision 1(6), 612-619.

Fitzpatrick M J, Starkschall G, Antolak J A, Fu J, Shukla H, Keall P J, Klahr P & Mohan R 2006 Displacement-based binning of time-dependent computed tomography image data sets Med Phys 33(1), 235-46.

Leng S, Zambelli J, Tolakanahalli R, Nett B, Munro P, Star-Lack J, Paliwal B & Chen G H 2008 Streaking artifacts reduction in four-dimensional cone-beam computed tomography Med Phys 35(10), 4649-59.

Li H, Noel C, Garcia-Ramirez J, Low D, Bradley J, Robinson C, Mutic S & Parikh P 2012 Clinical evaluations of an amplitude-based binning algorithm for 4DCT reconstruction in radiation therapy Med Phys 39(2), 922-32.

Lu J, Guerrero T M, Munro P, Jeung A, Chi P C, Balter P, Zhu X R, Mohan R & Pan T 2007 Four- dimensional cone beam CT with adaptive gantry rotation and adaptive data sampling Med Phys 34(9), 3520-9.

Mckinnon G C & Bates R H T 1981 Towards Imaging the Beating Heart Usefully with a Conventional Ct Scanner Ieee Transactions on Biomedical Engineering 28(2), 123-127.

O'Brien R T, Cooper B J & Keall P J 2013 Optimizing 4D cone beam computed tomography acquisition by varying the gantry velocity and projection time interval Phys Med Biol 58(6), 1705-23.

Roman N O, Shepherd W, Mukhopadhyay N, Hugo G D & Weiss E 2012 Interfractional positional variability of fiducial markers and primary tumors in locally advanced non-small-cell lung cancer during audiovisual biofeedback radiotherapy Int J Radiat Oncol Biol Phys 83(5), 1566-72.

Sidky E Y & Pan X 2008 Image reconstruction in circular cone-beam computed tomography by constrained, total-variation minimization Phys Med Biol 53(17), 4777-807.

Sonke J J, Zijp L, Remeijer P & van Herk M 2005 Respiratory correlated cone beam CT Medical Physics 32(4), 1176-1186.

Taguchi K 2003 Temporal resolution and the evaluation of candidate algorithms for four-dimensional CT Medical Physics 30(4), 640-650.

Talbi E G 2009 Metaheuristics: From Design to Implementation (Wiley Series on Parallel and Distributed Computing) John Wiley and Sons. Hoboken, New Jersey.

Zijp L, Sonke J & Herk M 2004 Extraction of the respiratory signal from sequential thorax cone-beam X-ray images. International conference on the Use of Computer in Radiation Therapy pp. 507- 509.

**BACKGROUND**

Any discussion of the background art throughout the specification should in no way be considered as an admission that such art is widely known or forms part of common general knowledge in the field.

Lung cancer is a leading cause of death worldwide. The survival rates are very low, even with treatment. There is an urgent need to improve survival rates. Radiation therapy can assist in treatment. An increase in tumor dose of 1-Gy results in a 4% improvement in survival. On the other hand, a 1-Gy decrease in overall mean lung dose results in a 2% reduction in pneumonitis.

Four dimensional cone beam computed tomography (4DCBCT) imaging is an emerging image guidance strategy used to position patients for treatment in radiotherapy. 4DCBCT was developed to overcome image blurring in 3DCBCT which is caused by respiratory motion and the long image acquisition times (several minutes). On the day of treatment, 4DCBCT provides valuable information on the average tumour position, the amplitude of the tumour motion, validation of the treatment plan and the changing tumour size and shape.

4DCBCT was first published between 2003 and 2005 (Taguchi 2003) and (Sonke et al. 2005) and commercially released by Elekta (Stockholm, Sweden) in 2009. 4DCBCT has been implemented on Elekta (Sonke et al. 2005), Varian (Lu et al. 2007) and Siemens (Dietrich et al. 2006) linear accelerators.

4DCBCT was developed to overcome image blurring in 3DCBCT which is caused by respiratory motion and the long image acquisition times (several minutes). On the day of treatment, 4DCBCT provides valuable information on the average tumour position, the amplitude of the tumour motion, validation of the treatment plan and the changing tumour size and shape.

CBCT (or 3D CBCT) imaging involves rotating a kilovoltage imager around a patient at a constant speed and acquiring 2D projections (kilovoltage images) with a constant time interval between projections. A series of evenly spaced projections are acquired around the patient's anatomy with a constant angular separation between projections. The series of 2D projections can be reconstructed into a 3D CBCT image using the Feldkamp-Davis-Kress (FDK) algorithms (Feldkamp et al. 1984).

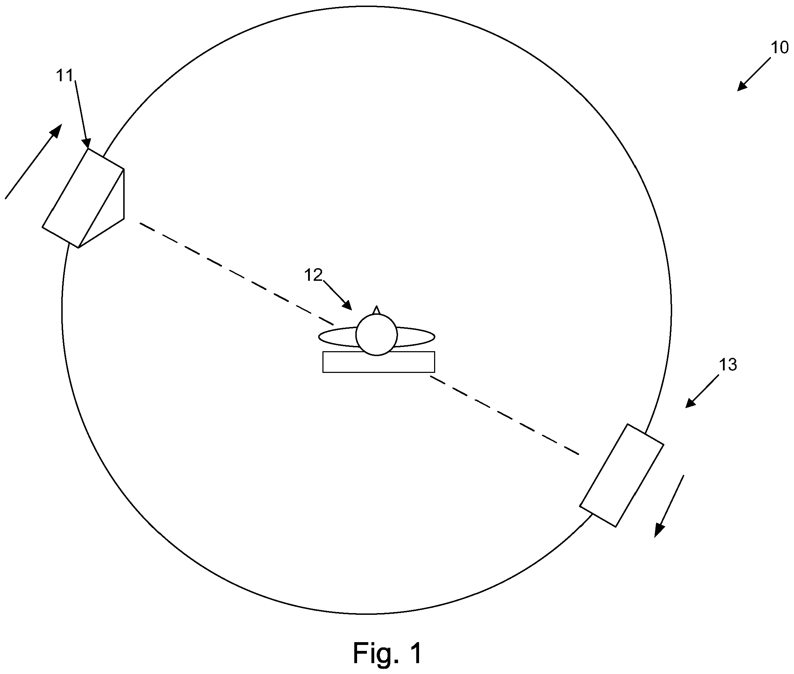

Fig. 1 illustrates schematically the operation of a 4D CBCT apparatus. In this arrangement, an emission source 11 is rotated around a patient 12 and Xrays are emitted. A detector 13 detects the beam attenuation by patient 12 and provides an output, which, subject to processing, provides an image data set for the patient.

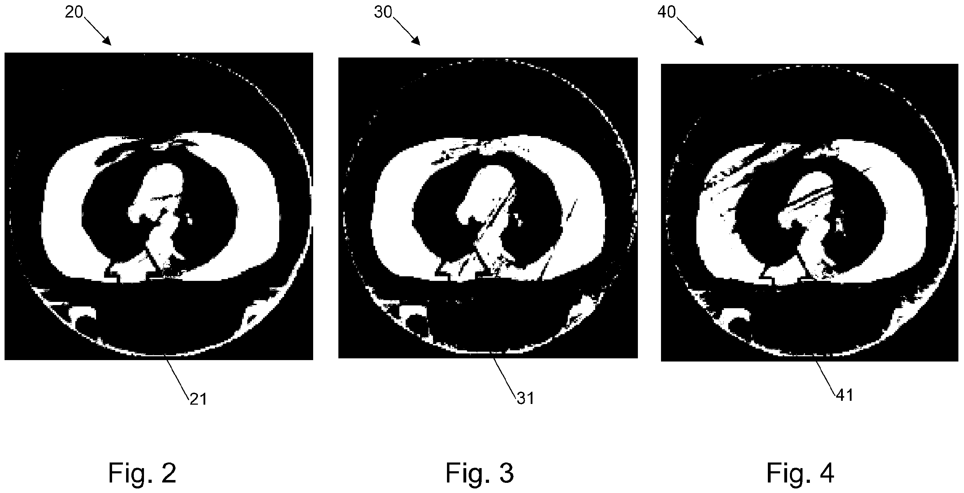

Fig. 2 to Fig. 4 illustrates and example of an acquired series of CBCT images. It can take several minutes to rotate the gantry around the patient in which time the patients anatomy moves due to respiratory motion. Fig. 2 illustrates a 3D CBCT image containing 2360 projections of a lung cancer patient. Fig. 3 illustrates a CBCT image in a peak inhale respiratory bin. Fig. 4 illustrates a CBCT image in the peak exhale respiratory bin. The arrow 21 points to a fiducial gold marker implanted in the tumour. The marker is blurred in the 3DCBCT image 20 and has moved 31, 41 between the peak exhale 30 and peak inhale 40 respiratory bins.

4DCBCT imaging differs from 3D CBCT imaging in that projections are allocated to different phases of the respiratory cycle and used to reconstruct an image for the corresponding respiratory phase. For example, projections acquired at peak exhale are allocated to a peak exhale respiratory bin and are used to reconstruct a 3D CBCT image in the peak exhale respiratory bin. Within each respiratory bin there is little anatomical motion and blurring artefacts are greatly reduced. The main problem with 4DCBCT imaging is streak artefacts which occur because a constant gantry speed and constant projection pulse rate are used (Leng et al. 2008).

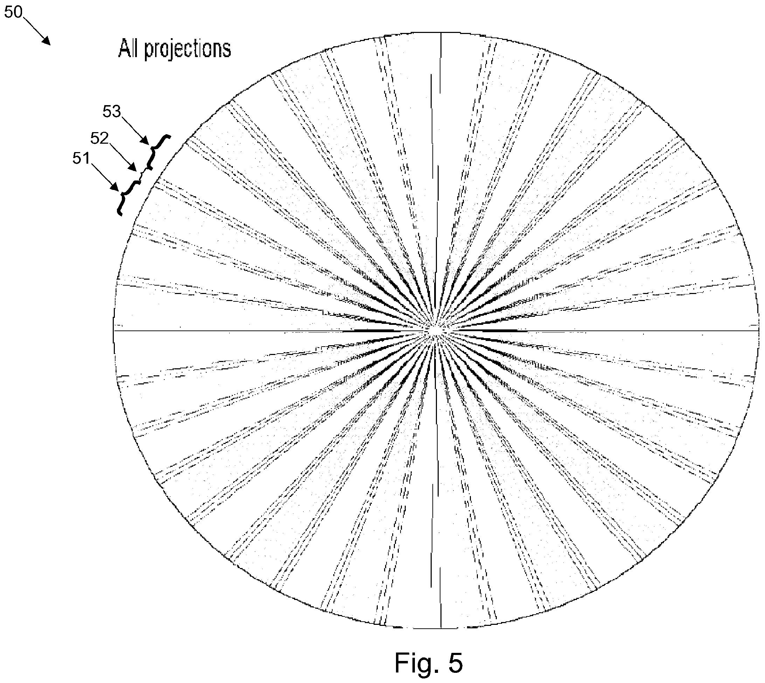

For example, as shown in Fig. 5, as the gantry is rotated around the patient at a constant speed, projections are acquired at a constant projection pulse rate. The figure illustrates all projections 50 acquired by rotating a gantry around a patient with an assumed constant repetitive sinusoidal breathing trace using a constant projection pulse rate and constant gantry speed. Projections taken during the top 1/3 inhale breathing 53, middle 1/3 of the breathing cycle 52 and the exhale portion of the breathing cycle 51 are illustrated. It will be noted that the middle of the breathing cycle includes a smaller interval. These intervals are repeated around the gantry rotation.

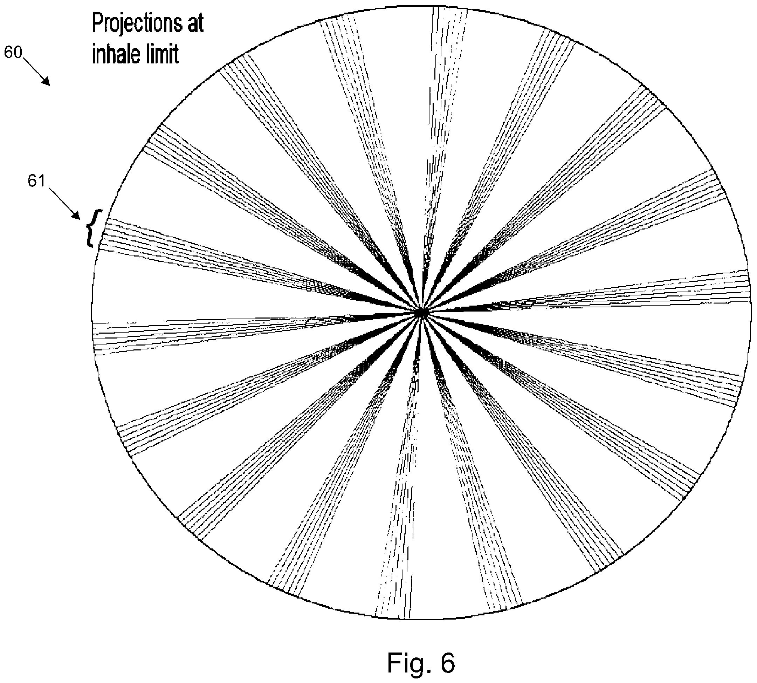

Fig. 6 illustrates 60 the projections acquired in the respiratory bin at peak exhale. These projections cluster e.g. 61 together whilst the patients breathing is in the peak exhale respiratory bin (typically about 0.4 seconds). Then a large angular gap between projections occurs as the gantry continues to move until the next respiratory signal 62 to re-enter the peak exhale respiratory bin (typically about 3.6 seconds).

In recent years there have been a number of studies with the focus on reducing the streak artefacts in 4DCBCT images. There are two common approaches taken: (1) A reconstruction approach where prior images, compressed sensing, iterative reconstruction, deformable image registration, etc, are used (Mckinnon & Bates 1981), (Leng et al. 2008), (Sidky & Pan 2008), (Chen et al. 2008) and (2) A hardware approach where the gantry speed or projection acquisition is modulated in order to evenly distribute projections in each respiratory bin (O'Brien et al. 2013), (Cooper et al. 2013) or (Fast et al. 2013). Both of these approaches will benefit if the streak artefacts in the CBCT images could be reduced or eliminated T. Li et al., "Enhanced 4D cone-beam CT with inter-phase motion model", Med. Phys 34(9), 3688-3695 discloses a 4DCBCT scan wherein respiratory bins are defined and their projections optimised using projection information from other phases after spatially registering that projection information using an inter-phase motion model.

It would be desirable if a there was a method that optimizes respiratory bin position, size and projection allocation in order to reduce streaking artefacts in 4DCBCT imaging.

## SUMMARY OF THE INVENTION

It is an object of the invention, in its preferred form to provide a method of optimizing 4D cone beam computed tomography (4DCBCT) images.

The invention is defined by claim 1. Further embodiments of the invention are defined by the dependent claims.

In some embodiments, the process involves reducing the variance of the angular separation between projections of each respiratory bin.

In some embodiments, the step (c) can comprise reducing an approximation to the standard deviation of the angular separation between projections of each respiratory bin.

In some embodiments, the step (c) preferably can include sharing projections between adjacent respiratory bins.

In some embodiments, the step (c) further can comprise limiting or expanding the size of each respiratory bin to a predetermined range.

In some embodiments, the step (c) preferably can include an iterative process of alteration of the boarders of each respiratory bin and recalculating the image quality measure to determine if a higher image quality can be provided.

In some embodiments, the number of projections within each of the respiratory bins can be able to increase or decrease by a predetermined amount.

According to one aspect of the disclosure, a simple heuristic is used to optimize the position of the respiratory bins. In a further aspect of the disclosure, projections from neighbouring respiratory bins can be used.

According to one aspect of the disclosure, the standard deviation (SD) is used to change the position of respiratory bins to optimize the position of the respiratory bins. In one aspect optimizing the position of respiratory bins includes operations that include minimizing the SD.

In another aspect of the disclosure, the standard deviation (SD) is used to determine if neighbouring projections can be shared.

## BRIEF DESCRIPTION OF THE DRAWINGS

Embodiments of the disclosure will now be described, by way of example only, with reference to the accompanying drawings in which:

Fig. 1 illustrates schematically a 4DCBCT image acquisition system;
Fig. 2 to Fig. 4 illustrates and example of an acquired series of CBCT images;
Fig. 5 illustrates schematically the operation of projection clustering;
Fig. 6 illustrates schematically projections at the inhale limit;
Fig. 7 illustrates the process of projection sharing;
Fig. 8 illustrates various transverse slices for a patient showing phase binning; and
Fig. 9 illustrates various transverse slices for a patient showing displacement binning.

## DETAILED DESCRIPTION

In the preferred embodiment a different approach is taken to the prior art in bin allocation as an attempt is made to make the best use of the projections acquired during 4DCBCT imaging by optimising the respiratory bin position, size and projection allocation. Although the projections in neighbouring bins are from a different part of the breathing phase, they contain valuable information about the patient's anatomy and can be used to reduce streaking artefacts. The result of this optimisation are 4DCBCT images with fewer streak artefacts and better image quality with the drawback that the centre of each respiratory bin is not always positioned in a predictable location.

During a 4DCBCT scan, the gantry moves with a slow constant speed while projections are acquired at a fixed rate (often around 11Hz). When a projection is acquired the respiratory signal, either displacement of the lungs or a respiratory phase, is also recorded. The respiratory signal, either displacement or phase, can be acquired either from an external sensor, such as the real-time position management system (RPM) from Varian Medical Systems, or from the images themselves (Zijp et al. 2004). If only the displacement of the lungs or tumour is recorded then the respiratory phase can be calculated by detecting two consecutive peak inhale points and then dividing the breathing cycle evenly in time with the phase ranging from 0 to $2\pi$ or 0% to 100%.

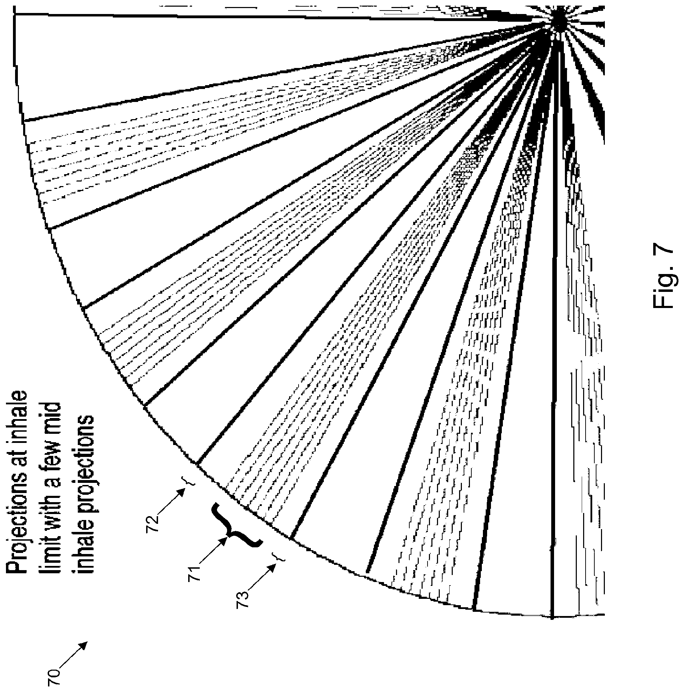

As illustrated in Fig. 7, in the preferred embodiments, for an example respiratory bin 71, a series of neighbouring projections on each side 72, 73 are utilised.

In the development of the embodiments of the disclosure, there is initially provided a full set of equations that represent the optimal respiratory bin and projection allocations. It has been found in practice that even with the state of the art Mixed Integer Programming (MIP) solvers, it is only possible to solve problems to optimality with 300 projections or less; which is well below practical problem sizes used for current 4DCBCT imaging. A simple heuristic solution method is then used to obtain a near optimal, but not provably optimal, solution to the MIP model. The simple heuristic greatly improves overall image quality. The equations presented apply to both phase based binning and displacement binning.

*Problem Formulation*

Let $r_j$ and $\theta_j$ be the respiratory signal (either phase or displacement) and gantry angle respectively for projection $j$. The projections are presumed to be sorted in order from the smallest gantry angle $j = 1$ to the largest gantry angle $j = N_p$ where $N_p$ is the number of projections.

*Respiratory bins:* To model the location of each respiratory bin, let Rb and Rb be the upper and lower respiratory signal respectively for respiratory bin $b$ ($b = 1, 2, ..., N_b$ with $N_b$ the number of respiratory bins). The values of Rb and Rb are to be determined as part of the optimisation but to make sure that the respiratory bins are contiguous the additional condition is that $R_b^u = R_{b+1}^l$ for $b = 2, 3, ... , N_b$, with $R_1^l = r_{min} = \min_j\{r_j\}$ and $R_{Nb}^u = r_{max} = max_j\{r_j\}$.

To ensure that respiratory bins do not become too large, and span a range of respiratory signals where significant anatomical motion takes place, it is necessary to place restrictions on the maximum size of each respiratory bin. If respiratory bins were evenly spaced then it would be expected the size of each respiratory bin, $(R_b^u - R_b^l)$, to be $\Delta = (r_{max} - r_{min})/ N_b$. In the preferred embodiments the respiratory bins are allowed to grow by a factor g or shrink by a factor s: i.e.: $\Delta (1 - s) \leq R_b^u - R_b^l \leq \Delta (1 + g)$ for $b = 1, 2, ... N_b$.

In our simulations, s = g = 0:5 which allows the respiratory bins to be 50% larger or smaller than $\Delta$. However, in practice it may be more practical to specify a fixed value in millimetres as the amount that the respiratory bins can grow, or shrink.

*Allocating projections to respiratory bins:* To determine if a projection is allocated to a respiratory bin, a binary variables $\delta_{b,j}$ is introduced which take the value 1 if projection j is allocated to respiratory bin b and zero otherwise. The values of $\delta_{b,j}$ are to be determined as part of the optimisation. The proximity constraints used to determine if the projection belongs to a respiratory bin are:

$$r_j \geq r_b^l \delta_{b,j} - \Delta_b^l \Delta, \qquad (1)$$

$$r_j \leq r_b^u + \Delta_b^u \Delta + r_{max} (1 - \delta_{b,j}), \quad (2)$$

for $b = 1, 2, ... , N_b$ and $j = 1, 2, ... , N_p$. The values of $\Delta_b^l$ and $\Delta_b^u$ are zero if sharing of projections between bins is not allowed, but can take a value if sharing of projection from neighbouring respiratory bins is allowed. If sharing of projections is allowed, projections were generally allowed to be taken from the neighbouring respiratory bin, so that $\Delta_b^l = \Delta_b^u = 1,$ but a value in millimetres may be more appropriate in practice. Investigation of values of $\Delta_b^l$ and $\Delta_b^u$ ranging from 0.5 to 1 were conducted in simulations.

To make sure that every projection is allocated to a respiratory bin we must have $\sum_b \delta_{b,j} = 1$ if projections cannot be shared between respiratory bins, and $\sum_b \delta_{b,j} \geq 1$ if projections can be shared between respiratory bins,

*The objective function:* An objective function that has been found to correlate well with image quality is the standard deviation (SD) of the angular separation between projections. For illustration purposes let $\theta_{b,k}$ be the $k^{th}$ largest gantry angle for the projections in respiratory bin $b$ ($k = 1, 2, ... , N_{p,b}$ where $N_{p,b}$ are the number of projections in respiratory bin $b$) then the SD in respiratory bin $b$ is

$$SD_b = \left[ \sum_{k=1}^{N_{p,b}-1} \left(\theta_{b,k+1} - \theta_{b,k} - \mu_b\right)^2 + \left(\theta_{b,1} + 2\pi - \theta_{b,N_{p,b}} - \mu_b\right)^2 \right]^{1/2}$$

where $\mu_b = 2\pi/N_{p,b}$ is the mean angular separation between projection in respiratory bin $b$. The SD for each respiratory bin can be averaged to give the average SD

$$SD = \sum_{b=1}^{N_b} SD_b / N_b \tag{3}$$

Equation 3 can be used when it is desired to calculate the SD from a given set of projections. Equation 3 is extensively used in the heuristic solution methods below. Unfortunately, it is not possible to minimise the SD in a linear optimisation algorithm because equation 3 contains quadratic terms and the value of $N_{p,b}$ is to be determined as part of the optimisation. However, equation 3 can be reduced down to linear terms which are suitable for use in mixed integer programming (MIP) solvers.

For example, the standard deviation in bin $b$ ($SD_b$) can be expressed in linear form as

$$SD_b = -4\pi^2 - 2\theta_{bsl} + 4\pi(\theta_{bs} - \theta_{bl}) - 2\pi\delta_{\theta,b} + \sum_j (2\delta b, j\, \theta j - 2\theta b, j),$$

with

$$\theta_{b,k} \leq M\delta_{b,k},$$

$$\theta_{b,k} \leq M\sum_{j>k} \delta b, j$$

$$\theta_{b,k} \leq M(2 - \delta_{b,k} - \delta_{b,j}) + \theta_k\theta_k',$$

$$\theta_{bl} \leq \theta_k + M\sum_{j>k} \delta b, j,$$

$$\theta_{bl} \geq \theta_k\delta_{b,k},$$

$$\theta_{bs} \leq \theta_k + M(1 - \delta_{b,k}),$$

$$\theta_{bs} \geq \theta_k - M\sum_{j<k} \delta b, j,$$

$$\theta_{bsl} \geq \theta_{bl}\theta_k + M(1 - \delta_{b,k}),$$

$$\theta_{bsl} \geq \theta_k - M\sum_{j<k} \delta b, j$$

$$\delta_{\theta,b} = 2\pi + \sum_j Cb, j$$

$$C_{b,k} \leq \delta_{\theta,b},$$

$$C_{b,k} \leq 2\pi\delta_{b,k},$$

$$C_{b,k} \geq \delta_{\theta,b} + M(\delta_{b,k} - 1),$$

for all $k$ and $k'$ and $M$ is a suitably large number.

*Constraints on the minimum and maximum SD:* In some cases the optimisation problem can reduce the SD in one respiratory bin at the cost of another respiratory bin. To ensure that each respiratory bin has enough projections, and a low enough SD to produce an image of suitable quality, a restriction is placed on the number of projections in each respiratory bin and the maximum SD:

$$SD_b \leq SD_{max} \text{ for all } b,$$

$$N_{min} \leq \sum_{j=1}^{N_{p,b}} \delta_{b,j} \leq N_{max} \text{ for all } b.$$

In the present implementation values of $SD_{max} = 2°$, $N_{min} = 120$ projections and $N_{max}$ is unlimited, were used.

**Method**

A solution to the optimisation problem presented above represents a good allocation of projections under the assumption that the SD is a good measure of image quality. However, even with the fastest commercial mixed integer programming solvers (XPRESS-MP, GUROBI and ILOG-CPLEX) it is difficult to solve problems with more than about 300 projections to optimality. For larger problems the computation time appears to grow exponentially with problem size and, in experiments, an optimal solution to a 1200 projection problem was not found within one month on a 16 Core 3.1Ghz machine.

*Heuristic Solution Method*

Although a provably optimal solution is difficult to obtain, considerable progress can be made using heuristic solution methods. Heuristic solution methods are used to obtain a good/near optimal solution in a small amount of time. There are a large range of heuristic solution methods available and each have their advantages and disadvantages (Talbi 2009). In alternative embodiments, a very simple heuristic solution method was used that obtains a rapid solution.

*The simple heuristic:* There are two steps in the simple heuristic. The first step involves optimising the location of the respiratory bins and the second step involves determining if projections in neighbouring respiratory bins will improve the SD.

**Step 1: The Respiratory Bin Position Algorithm**

(1) Move a respiratory bin a small increment either up or down in respiratory signal.

(2) Determine which projections fall within the new respiratory bins.

(3) Calculate the SD.

(4) If the SD is smaller than the best SD found then record the new respiratory bin locations as the best location.

(5) Systematically, move the respiratory bin again, or select a new respiratory bin. Go to step 1.

(6) Terminate the process if all possible respiratory bin locations have been exhausted.

The second step is to see if we can improve the SD by adding/sharing projections from neighbouring respiratory bins.

**Step 2: Projection Sharing Algorithm**

(1) For each respiratory bin find the candidate list of projections that are in a neighbouring respiratory bin and satisfy the proximity constraints (equations 1 and 2) which are a function of the parameters $\Delta_b^l$ and $\Delta_b^u$.

(2) Select a projection from the candidate list.

(3) Add the projection to the respiratory bin

(4) Calculate the SD with the new projection in the respiratory bin.

(5) If the SD is smaller than the best SD found permanently add the projection to the respiratory bin.

(6) Remove the projection from the candidate list and go to step 2.

(7) Terminate the process if we have no projections left in the candidate list.

*The extensive heuristic:* Applying the simple heuristic produces a good solution within 1 or 2 seconds of computation time and is a very useful algorithm to apply in practice. To determine if the simple heuristic produces a solution that is close to optimality we apply a more extensive heuristic that takes approximately 12 hours to determine if better solutions are available. The extensive heuristic is a simple extension on the simple heuristic and applies the projection sharing algorithm at each window position in the respiratory bin position optimisation algorithm. That is, the projection sharing algorithm is run at step 3 of the respiratory bin position algorithm.

*Patient Image Data*

Five 4DCBCT data sets from the study by (Roman et al. 2012) have been used. These 4DCBCT datasets were selected because the patients had fiducial gold coils implanted from which a respiratory signal can be extract. The marker around the thoracic cavity was used to extract the respiratory signal because it was the most easily segmented of the three markers. From the segmented marker a 3D trajectory was constructed and the superior-inferior (SI) component was used as the respiratory signal.

The five 4DCBCT datasets from the study of (Roman et al. 2012); were used. Two datasets from patient 1 and 2 and one dataset from the third patient. The five scans consist of 2360 (patient 1, image 1), 2406 (patient 1, image 2), 2508 (patient 2 image 1), 2435 (patient 2 image 2) and 2390 (patient 3 image 1) half fan projections respectively. For phase based binning, respiratory cycles were first identified by determining the peak exhalation points. The phase then rises linearly from 0 at peak exhale to 0.5 at peak inhale and then 1 at end exhale.

*Binning Options*

In all simulations a total of ten phase or displacement bins were used. The following binning options apply to both phase and displacement binning.

*1. Equispaced binning:* Respiratory cycles were divided into 10 equally spaced respiratory bins based on either phase or displacement. For displacement binning the peak inhale and exhale points were averaged to determine the location of the respiratory bins with any projections falling above or below the average points being allocated to the peak inhale or peak exhale respiratory bins respectively.

*2. Equal density binning:* The recorded respiratory signals for each projection were sorted from lowest to highest. The lowest 10% were allocated to the first respiratory bin, the second 10% were allocated to the second respiratory bin and so on. This method ensures that every bin has exactly the same number of projections but the size and location of each respiratory bin cannot be controlled.

*3. Optimized respiratory bins:* Both the simple$_b$ and extensive heuristic have been used to determine the location of projections and the allocation of projections to respiratory bins. In the optimisation the respiratory bins were allowed to grow, or shrink, by 50%, and projections could be sourced from either half way $(\Delta_b^l = \Delta_b^u = 0.5)$ or from the whole neighbouring respiratory bin $(\Delta_b^l = \Delta_b^u = 1)$. A minimum of 120 projections per respiratory bin were required to ensure that image quality is adequate.

*Image Reconstruction*

Images were reconstructed using COBRA to give 96 transverse slices of dimension 224×224 pixels. Each slice in the reconstructed image was 2mm and the voxel size was 2mm×2mm×2mm.

*Marker Size Estimation*

To determine if sourcing projections from neighbouring respiratory bins has blurred the image we segmented one fiducial gold marker per dataset and calculated the volume of the marker in each respiratory bin. If significant blurring of the marker has taken place then we expect the volume of the marker to be inconsistent across the respiratory bins. The fiducial gold coils were 0.35mm× 10mm or 20mm in length (Visicoil, RadioMed Corp., Tynsboro, MA) (Roman et al. 2012). To calculate the size of the gold coils from the reconstructed 4DCBCT images, we select a region around the coils of 30mm×30mm×24mm in the lateral, anterior-posterior and superior-inferior directions respectively. We compute the mean and standard deviation of the pixels within the region. Pixels with an intensity of two standard deviations above the mean were selected as candidate pixels. From the candidate pixels the largest connected cluster of pixels were selected as the marker. We then calculate the centre-of-mass (COM) of the marker to represent the location of the marker.

## 4. Results

There are three different parameters measured in the results:

(1) reconstructed images, (2) the SD of the angular separation between projections and (3) an analysis of the consistency of the marker size segmented in the reconstructed images.

*Reconstructed Images*

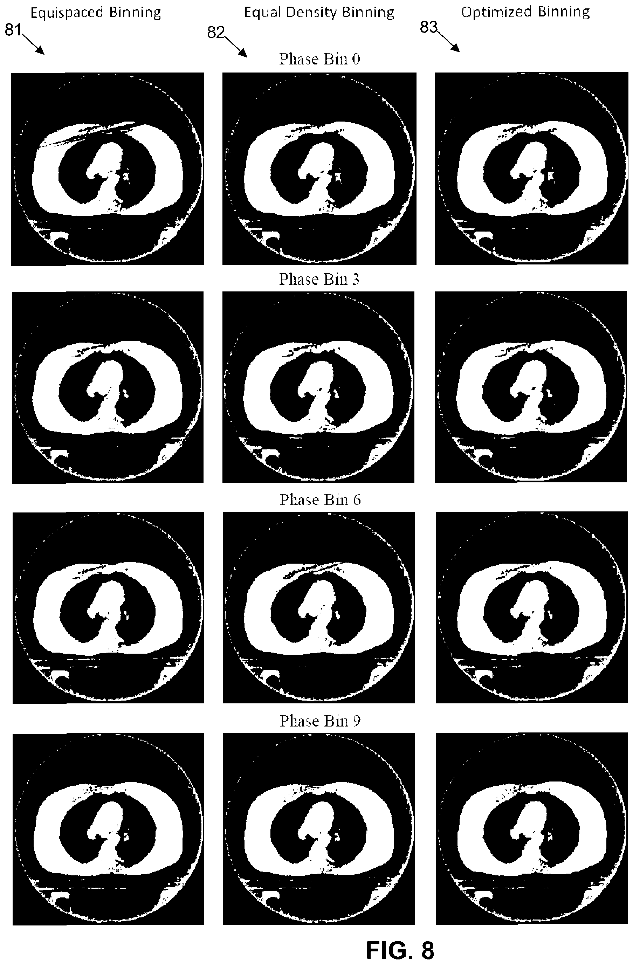

A transverse slice of patient 1 image 1 was selected showing the location of the fiducial gold marker. Fig. 8 illustrates images of the image data for patient 1 image 1 for phase based binning using equispaced 81, equal density 82 and optimized binning 83. Optimized binning was found to produce better, or the same quality, images in all respiratory bins than both equispaced or equal density binning. For bin 0 with equispaced binning and bin 6 with equal density binning, the optimized binning option was able to eliminate the dominant streak artefacts observed.

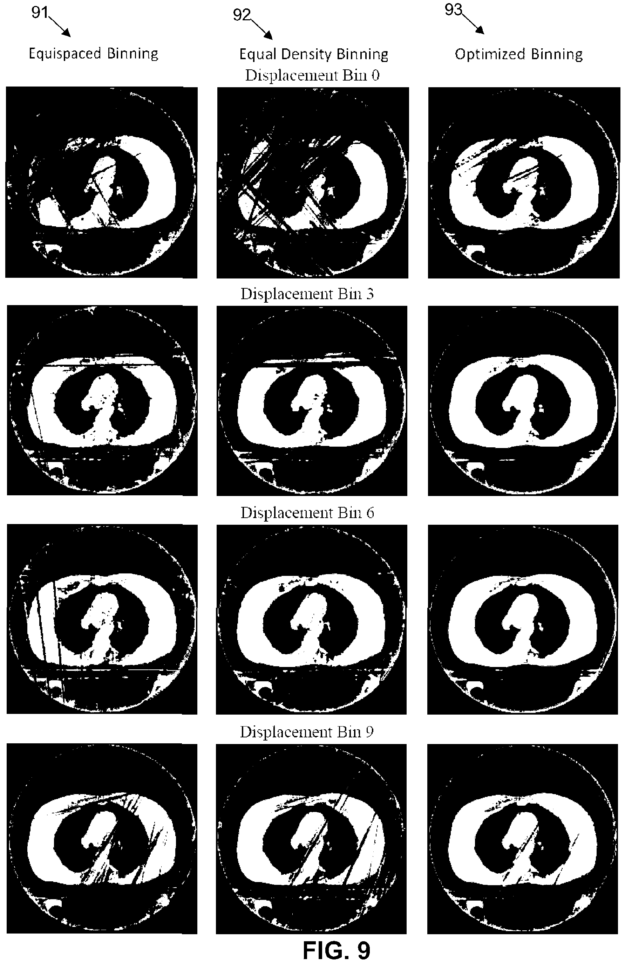

Fig. 9 gives images of the data for patient 1 image 1 for displacement binning using equispaced 91, equal density 92 and optimized binning 93. Rows one, two, three and four are for respiratory bin 0, 3, 6 and 9 respectively. Optimized binning allowed projections to be sourced from half way into the neighbouring respiratory bin and a minimum of 120 projections must be used per respiratory bin. With optimized binning the streak artefacts are significantly reduced and image quality is significantly better. Optimized binning produces better, or the same quality, images in all respiratory bins than both equispaced or equal density binning. For displacement binning the image quality is greatly improved using optimized binning. Both streak artefacts and image sharpness are better. In the peak inhale and peak exhale respiratory bins, although the streaking artefacts have been reduced with optimized binning, there are still some streak artefacts. These can be reduced further by allowing the respiratory bins to grow by more than 50% with the downside that image blurring is likely to increase due to the larger range of motion in the peak exhale and peak inhale respiratory bins.

*The SD Between Projections*

As the purpose of the optimisation is to reduce the SD of the angular separation between projections it is important to establish how much we can reduce the SD. In table 1 there is shown the SD for the five 4DCBCT datasets using equispaced, equal density and optimized binning. The results presented examine$_b$ the influence of the sharing distance parameters ( $\Delta_b^u$ and $\Delta_b^l$ ) and the minimum number of projections per respiratory bin on the SD. The table shows the SD (equation 3) of the angular separation between projections. The sharing distance parameters ( $\Delta_b^u$ and $\Delta_b^l$ ) can be either 0.5 or 1.0 allowing projections to be sourced from half way and the full neighbouring respiratory bin respectively. Foreign projections are the percentage of projections that have been sourced from the neighbouring respiratory bin.

Table 1

| Patient Scan | $\Delta^l$/ $\Delta^u$ | Equispac ed SD | Equal Density SD | Simple Heuristic | | | Extensive Heuristic | | |
|---|---|---|---|---|---|---|---|---|---|
| | | | | SD | Total Proj's | Foreign Proj's | SD | Total Proj's | Foreign Proj's |
| Phase Based Binning | | | | | | | | | |
| 1-1 | 0.5 | 1.2° | 1.3° | 0.9° | 2866 | 17.7% | 0.9° | 2873 | 17.9% |
| 1-2 | 0.5 | 1.6° | 1.3° | 0.9° | 3016 | 20.2% | 0.8° | 2902 | 16.4% |
| 2-1 | 0.5 | 1.6° | 1.5° | 1.2° | 3106 | 19.2% | 1.2° | 3020 | 16.9% |
| 2-2 | 0.5 | 1.5° | 1.5° | 1.2° | 3024 | 18.8% | 1.2° | 2987 | 18.5% |
| 3-1 | 0.5 | 1.8° | 1.8° | 1.6° | 3027 | 20.0% | 1.5° | 2949 | 19.0% |
| Phase Based Binning | | | | | | | | | |
| 1-1 | 1.0 | 1.2° | 1.3° | 0.9° | 2995 | 21.2% | 0.8° | 2873 | 22.6% |
| 1-2 | 1.0 | 1.6° | 1.3° | 0.8° | 3236 | 25.7% | 0.8° | 3258 | 25.3% |
| 2-1 | 1.0 | 1.6° | 1.5° | 1.1° | 3412 | 25.9% | 1.2° | 3224 | 22.2% |
| 2-2 | 1.0 | 1.5° | 1.5° | 1.1° | 3451 | 28.8% | 1.1° | 3581 | 31.7% |
| 3-1 | 1.0 | 1.8° | 1.8° | 1.5° | 3097 | 22.7% | 1.4° | 3248 | 26.4% |
| Displacement Binning | | | | | | | | | |
| 1-1 | 0.5 | 2.6° | 3.9° | 0.8° | 3634 | 35.1% | 0.7° | 3746 | 37.0% |
| 1-2 | 0.5 | 2.2° | 3.1° | 1.1° | 3872 | 37.8% | 1.0° | 3713 | 35.2% |
| 2-1 | 0.5 | 2.5° | 2.4° | 1.0° | 4683 | 46.4% | 0.9° | 4655 | 46.1% |
| 2-2 | 0.5 | 2.2° | 4.3° | 0.8° | 3966 | 38.6% | 0.7° | 4240 | 42.5% |
| 3-1 | 0.5 | 2.0° | 3.2° | 0.8° | 3835 | 37.7% | 0.8° | 3901 | 38.8% |
| Displacement Binning | | | | | | | | | |
| 1-1 | 1.0 | 1.9° | 1.9° | 0.6° | 4732 | 50.1% | 0.6° | 3746 | 48.7% |
| 1-2 | 1.0 | 2.2° | 3.1° | 0.7° | 5150 | 53.3% | 0.7° | 5361 | 55.1% |
| 2-1 | 1.0 | 2.5° | 2.4° | 0.6° | 6317 | 60.1% | 0.6° | 6206 | 59.6% |
| 2-2 | 1.0 | 2.2° | 4.3° | 0.6° | 4755 | 48.8% | 0.5° | 4955 | 50.8% |
| 3-1 | 1.0 | 2.0° | 3.2° | 0.7° | 4535 | 47.3% | 0.7° | 4292 | 44.3% |

In all cases, the SD is reduced using optimized binning when compared to either equispaced and equal density binning. The improvement in the SD is more significant for displacement binning which is not unexpected as phase based binning was designed to overcome the data sufficiency problems with displacement binning. For displacement binning, the SD is more than halved for all datasets using all parameter settings. For phase based binning the improvement in the SD is usually in the range of 10-30%.

With phase based binning, the number of projections sourced from neighbouring respiratory bins ranges from 17.7% to 30.6%. For displacement binning a lot more projections are sourced from neighbouring respiratory bins with the number ranging from 30% to 50.6%. As expected the number of projections sourced from neighbouring respiratory bins is higher when projections are sourced from the entire neighbouring respiratory bin compared to only half way into the neighbouring respiratory bin.

Comparing the simple and extensive heuristic gives an indication on the quality of the simple heuristic. For phase based binning the extensive heuristic was able to find significantly better solutions, while for displacement binning the extensive heuristic did not find significantly better solutions. The implication is that development of a fast heuristic for phase based binning has the potential to improve image quality.

*The Consistency of the Segmented Marker Volume*

Table 2 below lists the segmented marker volume in pixels for the five 4DCBCT datasets with projection sharing from the entire neighbouring respiratory bin. Segmented marker volumes. The mean and standard deviation across the 10 respiratory bins is given in pixels for the cases where segmentation was successful. Failed is the number of respiratory bins (maximum 10) for which the segmented location had an error greater than 3mm. The 3DCBCT image row used all projections to generate a single CBCT image. Motion of the marker during image acquisition results in significant blurring of the marker leading to a larger, but less intense, marker. As a result the 3D CBCT images have fewer pixels above the mean plus two standard deviation threshold for a pixel to be counted as a marker and therefore have a lower number of pixels detected.

Table 2

| Binning Method | Phase Binning | | | Displacement Binning | | |
|---|---|---|---|---|---|---|
| | Failed | Mean | Standard Deviation | Failed | Mean | Standard Deviation |
| **Patient 1 Image 1** | | | | | | |
| Equispaced | 0 | 20 | 2.1 | 2 | 27 | 18.1 |
| Equal-Density | 0 | 21 | 1.8 | 2 | 32 | 26.8 |
| Optimized | 0 | 20 | 2.6 | 0 | 19 | 1.7 |
| 3D CBCT | | 14 | | | 14 | |
| **Patient 1 Image 2** | | | | | | |
| Equispaced | 0 | 25 | 7.4 | 4 | 47 | 30.5 |
| Equal-Density | 0 | 22 | 3.0 | 2 | 41 | 24.0 |
| Optimized | 0 | 20 | 3.6 | 0 | 22 | 5.2 |
| 3D CBCT | | 23 | | | 23 | |
| **Patient 2 Image 1** | | | | | | |
| Equispaced | 3 | 36 | 10.4 | 3 | 33 | 15.9 |
| Equal-Density | 4 | 35 | 9.3 | 6 | 27 | 18.9 |
| Optimized | 2 | 37 | 7.8 | 0 | 41 | 7.0 |
| 3D CBCT | | 44 | | | 44 | |
| **Patient 2 Image 2** | | | | | | |
| Equispaced | 0 | 15 | 2.6 | 1 | 27 | 38.7 |
| Equal-Density | 0 | 15 | 1.9 | 1 | 14 | 4.2 |
| Optimized | 0 | 15 | 2.2 | 0 | 16 | 1.3 |
| 3D CBCT | | 16 | | | 16 | |
| **Patient 3 Image 1** | | | | | | |
| Equispaced | 0 | 16 | 10.5 | 1 | 11 | 2.4 |
| Equal-Density | 0 | 13 | 4.3 | 1 | 13 | 6.0 |
| Optimized | 0 | 13 | 6.1 | 0 | 12 | 1.2 |
| 3D CBCT | | 13 | | | 13 | |

The phase based binning results produce consistent marker sizes between the three binning methods for all five datasets. There is only one dataset where the optimized binning produces the lowest standard deviation of the three methods with the standard deviation being close to the best binning method for the four other datasets. This clearly indicates that there is a measurable, but generally insignificant, amount of blurring of the marker using optimized binning. It should be noted that optimized binning eliminates the bad results (for example patient 2 image 1 and patient 3 image 1 with equispaced binning).

The displacement binning results indicate that optimized binning is able to segment the marker in all cases while equispaced and equal-density binning have a large number of failed segmentations (usually at inhale and exhale limits). The standard deviation for optimized binning is always lower than equispaced and equal-density binning. Displacement binning, with the optimization algorithm, produces a lower standard deviation in marker volumes than phase based binning for all datasets except for patient 1 image 2. This indicates that on average the marker can be more consistently segmented using displacement binning than phase binning.

Significant improvements in image quality have been observed using displacement binning with the optimized binning algorithm. In clinical practice phase based binning is usually preferred because of data sufficiency problems and streak artefacts present in the displacement binned reconstructed images. However, it has been demonstrated that displacement binning is more accurate, contains less motion artefacts and recovers the tumour size and shape better than phase binning for 4DCT (Abdelnour et al. 2007), (Fitzpatrick et al. 2006), (Li et al. 2012). Our algorithms present a pathway to reliably generate streak reduced displacement based 4DCBCT images.

Streaking artefacts significantly degrade the quality of deformable image registration. Applications using deformable image registration, such as lung ventilation studies, are likely to benefit using these algorithms. Additionally, several iterative reconstruction techniques utilise deformable image registration either between prior images or between respiratory bins. These image registration techniques are also likely to benefit from the binning approach.

Of course, heuristic based methods suffer from their own issues. From an optimisation point of view solving the equations to optimality would help to benchmark the faster, but not guaranteed optimal, heuristic methods.

Sourcing projections from a large distance into the neighbouring bins is likely to produce images with less streaks but the trade off is that there will be more respiratory motion present in the projections which could potentially produce more blurring artefacts. From a clinical point of view it is beneficial if respiratory bins are located at a predictable location so deformable image registration may be necessary to recover images with a uniform spacing. With better image quality there is the potential for reducing both imaging time and imaging dose (less projections). The binning algorithms open the door for the potential clinical use of displacement binning which will improve image quality and patient positioning in radiotherapy.

In summary, four dimensional cone beam computed tomography (4DCBCT) is an emerging image guidance strategy used in radiotherapy where projections acquired during a 4DCBCT scan are sorted into respiratory bins based on the respiratory phase or displacement. However, 4DCBCT images suffer from streaking artefacts as a result of under sampling due to the long scan times, the irregular nature of patient breathing and the binning algorithms used. For displacement binning, the streaking artefacts are so severe that displacement binning is rarely used clinically. In the preferred embodiment there is provided a method of sharing projections between respiratory bins and adjusting the location of respiratory bins to reduce or eliminate streaking artefacts in 4DCBCT images. Five 4DCBCT datasets from three patients were used to reconstruct 4DCBCT images. Projections were sorted into respiratory bins using equispaced, equal density and projection sharing. The standard deviation (SD) of the angular separation between projections and the consistency of the segmented volume of a fiducial gold coil were assessed from the reconstructed images. Using the disclosed projection sharing methods, the SD of the angular separation between projections was reduced by more than 50% with displacement binning and between 10-30% with phase binning. Images reconstructed using displacement binning and the projection sharing algorithm were clearer, contained significantly less streak artefacts and allowed more consistent marker segmentation than those reconstructed with either equispaced or equal-density binning. Images reconstructed using phase binning and projection sharing were as good or better than those obtained using either equispaced or equal density binning. The volume of the marker segmented in the reconstructed images was more consistent using projection sharing and displacement binning than any phase binning approach. The projection sharing approach significantly improves image quality in 4DCBCT images and allows for the potential use of displacement binning in clinical applications.

**Interpretation**

Reference throughout this specification to "one embodiment", "some embodiments" or "an embodiment" means that a particular feature, structure or characteristic described in connection with the embodiment is included in at least one embodiment of the present disclosure. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

In the claims below and the description herein, any one of the terms comprising, comprised of or which comprises is an open term that means including at least the elements/features that follow, but not excluding others. Thus, the term comprising, when used in the claims, should not be interpreted as being limitative to the means or elements or steps listed thereafter. For example, the scope of the expression a device comprising A and B should not be limited to devices consisting only of elements A and B. Any one of the terms including or which includes or that includes as used herein is also an open term that also means including at least the elements/features that follow the term, but not excluding others. Thus, including is synonymous with and means comprising.

As used herein, the term "exemplary" is used in the sense of providing examples, as opposed to indicating quality. That is, an "exemplary embodiment" is an embodiment provided as an example, as opposed to necessarily being an embodiment of exemplary quality.

It should be appreciated that in the above description of exemplary embodiments of the disclosure, various features of the disclosure are sometimes grouped together in a single embodiment, FIG., or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment.

Furthermore, while some embodiments described herein include some but not other features included in other embodiments, combinations of features of different embodiments are meant to be within the scope of the disclosure, and form different embodiments, as would be understood by those skilled in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

Similarly, it is to be noticed that the term coupled, when used in the claims, should not be interpreted as being limited to direct connections only. The terms "coupled" and "connected," along with their derivatives, may be used. It should be understood that these terms are not intended as synonyms for each other. Thus, the scope of the expression a device A coupled to a device B should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still cooperate or interact with each other.

## Claims

1. A method of reducing artefacts in image creation in 4D cone beam computed tomography (4DCBCT) images, the method comprising the steps of:

   (a) performing a 4DCBCT scan (10) of a target patient including a series of spaced apart projections (50, 60, 70) through the target patient, with each projection having an associated estimated or measured respiratory state;
   (b) initially dividing the series of projections into a corresponding series of respiratory bins (71), with each respiratory bin having projections substantially from a portion of a cyclic respiratory state;
   (c) optimising the projections at the bounds of each respiratory bin (72, 73) so as to improve an image quality measure of the images wherein said optimising includes sharing projections between adjacent respiratory bins.

2. A method as claimed in claim 1 where said image quality measure includes utilising a measure of the variance of the angular separation between projections of each respiratory bin.

3. A method as claimed in claim 1 wherein said step (c) comprises reducing an approximation to the standard deviation of the angular separation between projections of each respiratory bin.

4. A method as claimed in claim 1 wherein said step (c) includes an iterative process of alteration of the borders of each respiratory bin and recalculating the image quality measure to determine if a better image quality measure is provided.

5. A method as claimed in any previous claim wherein the number of projections within each of said respiratory bins is able to increase or decrease by a predetermined amount.

**Patentansprüche**

1. Verfahren zum Reduzieren von Artefakten bei der Bilderzeugung in 4D-Kegelstrahl-Computertomographie (4DCBCT) -Bildern, wobei das Verfahren die folgenden Schritte aufweist:

   (a) Durchführen eines 4DCBCT-Scans (10) eines Zielpatienten mit einer Reihe voneinander beabstandeter Projektionen (50, 60, 70) durch den Zielpatienten, wobei jede Projektion einen zugehörigen geschätzten oder gemessenen Atmungszustand aufweist;
   (b) anfängliches Aufteilen der Reihe von Projektionen in eine entsprechende Reihe von Atmungsfächern (71), wobei jedes Atmungsfach Projektionen im Wesentlichen von einem Teil eines zyklischen Atmungszustands hat;
   (c) Optimieren der Projektionen an den Grenzen jedes Atmungs-Fachs (72, 73), um ein Bildqualitätsmaß der Bilder zu verbessern, wobei das Optimieren das Teilen von Projektionen zwischen benachbarten Atmungsfächern beinhaltet.

2. Verfahren gemäß Anspruch 1, wobei das Bildqualitätsmaß ein Verwenden eines Maßes der Varianz des Winkelabstandes zwischen Projektionen jedes Atmungsfaches umfasst.

3. Verfahren gemäß Anspruch 1, wobei der Schritt (c) das Reduzieren einer Abschätzung der Standardabweichung des Winkelabstandes zwischen Projektionen jedes Atmungsfaches umfasst.

4. Verfahren gemäß Anspruch 1, wobei der Schritt (c) einen iterativen Prozess der Änderung der Grenzen jedes Atmungsfaches und des Neuberechnens des Bildqualitätsmaßes beinhaltet, um zu bestimmen, ob ein besseres Bildqualitätsmaß bereitgestellt wird.

5. Verfahren gemäß einem der vorhergehenden Ansprüche, wobei die Anzahl der Projektionen in jedem der Atmungsfächer um einen vorbestimmten Betrag erhöht oder verringert werden kann.

**Revendications**

1. Procédé de réduction d'artefacts lors d'une création d'image sur des images de tomographie calculées par un faisceau de cône en 4D (4DCBCT), le procédé comprenant les étapes qui consistent à :

   (a) effectuer un balayage 4DCBCT (10) d'un patient cible comprenant une série de projections espacées (50, 60, 70) à travers le patient cible, chaque projection ayant un état respiratoire associé estimé ou mesuré ;
   (b) diviser initialement la série de projections en une série correspondante de sections respiratoires (71), chaque section respiratoire ayant des projections sensiblement depuis une partie d'un état respiratoire cyclique ;
   (c) optimiser les projections aux limites de chaque section respiratoire (72, 73) de façon à améliorer une mesure de qualité d'image des images, ladite optimisation comprenant le partage de projections entre des sections respiratoires adjacentes.

2. Procédé selon la revendication 1, dans lequel ladite mesure de qualité d'image comprend l'utilisation d'une mesure de la variance de la séparation angulaire entre les projections de chaque section respiratoire.

3. Procédé selon la revendication 1, dans lequel ladite étape (c) comprend la réduction d'une approximation à l'écart-type de la séparation angulaire entre les projections de chaque section respiratoire.

4. Procédé selon la revendication 1, dans lequel ladite étape (c) comprend un processus répétitif de modification des limites de chaque section respiratoire et de recalcul de la mesure de qualité d'image afin de déterminer si une meilleure mesure de qualité d'image est obtenue.

5. Procédé selon l'une quelconque des revendications précédentes, dans lequel le nombre de projections dans chacune desdites sections respiratoires peut augmenter ou diminuer selon une quantité prédéterminée.

Fig. 1

Fig. 4

Fig. 3

Fig. 2

Fig. 5

Projections at inhale limit

Fig. 6

Projections at inhale
limit with a few mid
inhale projections

70

72

71

73

Fig. 7

EP 3 102 111 B1

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ABDELNOUR A F ; NEHMEH S A ; PAN T ; HUMM J L ; VERNON P ; SCHODER H ; ROSENZWEIG K E ; MAGERAS G S ; YORKE E ; ARSON S M.** Phase and amplitude binning for 4D-CT imaging. *Phys Med Biol,* 2007, vol. 52 (12), 3515-29
- **CHEN G H ; TANG J ; LENG S.** Prior image constrained compressed sensing (PICCS): a method to accurately reconstruct dynamic CT images from highly undersampled projection data sets. *Med Phys,* 2008, vol. 35 (2), 660-3
- **COOPER B J ; O'BRIEN R T ; BALIK S ; HUGO G D ; KEALL P J.** Respiratory triggered 4D cone-beam computed tomography: a novel method to reduce imaging dose. *Med Phys,* 2013, vol. 40 (4), 041901
- **DIETRICH L ; JETTER S ; TUCKING T ; NILL S ; OELFKE U.** Linac-integrated 4D cone beam CT: first experimental results. *Phys Med Biol,* 2006, vol. 51 (11), 2939-52
- **FAST M ; WISOTZKY E ; OELFKE U ; NILL S.** *Actively Triggered 4d Cone-Beam CT Acquisition Medical Physics,* 2013, vol. 40 (9
- **FELDKAMP L A ; DAVIS L C ; KRESS J W.** *Practical Cone-Beam Algorithm Journal of the Optical Society of America a-Optics Image Science and Vision,* 1984, vol. 1 (6), 612-619
- **FITZPATRICK M J ; STARKSCHALL G ; ANTOLAK J A ; FU J ; SHUKLA H ; KEALL P J ; KLAHR P ; MOHAN R.** Displacement-based binning of time-dependent computed tomography image data sets. *Med Phys,* 2006, vol. 33 (1), 235-46
- **LENG S ; ZAMBELLI J ; TOLAKANAHALLI R ; NETT B ; MUNRO P ; STAR-LACK J ; PALIWAL B ; CHEN G H.** Streaking artifacts reduction in four-dimensional cone-beam computed tomography. *Med Phys,* 2008, vol. 35 (10), 4649-59
- **LI H ; NOEL C ; GARCIA-RAMIREZ J ; LOW D ; BRADLEY J ; ROBINSON C ; MUTIC S ; PARIKH P.** Clinical evaluations of an amplitude-based binning algorithm for 4DCT reconstruction in radiation therapy. *Med Phys,* 2012, vol. 39 (2), 922-32
- **LU J ; GUERRERO T M ; MUNRO P ; JEUNG A ; CHI P C ; BALTER P ; ZHU X R ; MOHAN R ; PAN T.** Four- dimensional cone beam CT with adaptive gantry rotation and adaptive data sampling. *Med Phys,* 2007, vol. 34 (9), 3520-9
- **MCKINNON G C ; BATES R H T.** *Towards Imaging the Beating Heart Usefully with a Conventional Ct Scanner Ieee Transactions on Biomedical Engineering,* 1981, vol. 28 (2), 123-127
- **O'BRIEN R T ; COOPER B J ; KEALL P J.** Optimizing 4D cone beam computed tomography acquisition by varying the gantry velocity and projection time interval. *Phys Med Biol,* 2013, vol. 58 (6), 1705-23
- **ROMAN N O ; SHEPHERD W ; MUKHOPADHYAY N ; HUGO G D ; WEISS E.** Interfractional positional variability of fiducial markers and primary tumors in locally advanced non-small-cell lung cancer during audiovisual biofeedback radiotherapy. *Int J Radiat Oncol Biol Phys,* 2012, vol. 83 (5), 1566-72
- **SIDKY E Y ; PAN X.** Image reconstruction in circular cone-beam computed tomography by constrained, total-variation minimization. *Phys Med Biol,* 2008, vol. 53 (17), 4777-807
- **SONKE J J ; ZIJP L ; REMEIJER P ; HERK M.** Respiratory correlated cone beam. *CT Medical Physics,* 2005, vol. 32 (4), 1176-1186
- **TAGUCHI K.** Temporal resolution and the evaluation of candidate algorithms for four-dimensional. *CT Medical Physics,* 2003, vol. 30 (4), 640-650
- **TALBI E G.** Metaheuristics: From Design to Implementation. Wiley Series on Parallel and Distributed Computing, 2009
- **ZIJP L ; SONKE J ; HERK M.** Extraction of the respiratory signal from sequential thorax cone-beam X-ray images. *International conference on the Use of Computer in Radiation Therapy,* 2004, 507-509
- **T. LI et al.** Enhanced 4D cone-beam CT with inter-phase motion model. *Med. Phys,* vol. 34 (9), 3688-3695